# EUROPÄISCHE PATENTSCHRIFT

(11) **EP 4 011 385 B1**
(45) Veröffentlichungstag und Bekanntmachung des Hinweises auf die Patenterteilung: **29.04.2026**
(21) Anmeldenummer: 21212995.1
(22) Anmeldetag: 07.12.2021
(51) Int. Cl.: A61K 36/00, A61P 15/00, A61P 15/14, A61P 31/04, A61K 36/67, A61K 36/9066, A61K 31/05, A61K 31/08, A61K 31/12, A61K 31/4525

(54) **WIRKSTOFF ZUR BEHANDLUNG DER MASTITIS**
ACTIVE SUBSTANCE FOR THE TREATMENT OF MASTITIS
SUBSTANCE ACTIVE POUE TRAITEMENT DES MAMMITES

(43) Veröffentlichungstag der Anmeldung: 15.06.2022
(73) Patentinhaber: Becker, Diko Holger, 46414 Rhede Krommert (DE)
(72) Erfinder: Becker, Diko Holger, 46414 Rhede Krommert (DE)
(74) Vertreter: Kayser, Christoph

(56) Entgegenhaltungen:
- WO-A1-2016/097755
- WO-A1-97/01348
- WO-A1-97/07812
- FRATINI FILIPPO ET AL: "Antibacterial activity of essential oils, their blends and mixtures of their main constituents against some strains supporting livestock mastitis", FITOTERAPIA, IDB HOLDING, MILAN, IT, vol. 96, 13 April 2014 (2014-04-13), pages 1 - 7, XP028873716, ISSN: 0367-326X, DOI: 10.1016/J.FITOTE.2014.04.003
- MUSHTAQ SALEEM ET AL: "Bovine mastitis: An appraisal of its alternative herbal cure", MICROBIAL PATHOGENESIS, ACADEMIC PRESS LIMITED, NEW YORK, NY, US, vol. 114, 9 December 2017 (2017-12-09), pages 357 - 361, XP085349481, ISSN: 0882-4010, DOI: 10.1016/J.MICPATH.2017.12.024

## Beschreibung

Die vorliegende Erfindung betrifft einen Wirkstoff zur Verwendung für die Behandlung von Mastitis.

Bei der Mastitis handelt es sich um eine Entzündung der Brustdrüse beziehungsweise der Milchdrüse bei Säugern. Mastitis kann im Prinzip bei allen Säugetieren auftreten. Besonders häufig finden sich Euterentzündungen beim Menschen, Wiederkauern, Pferden und Schweinen.

Beispielsweise kann Mastitis in Mastsäuen zu Unterernährung und erhöhter Sterblichkeit der Ferkel führen und kann sich kurz- und langfristig negativ auf das Wachstum und die Gesundheit der Sau und der Ferkel auswirken.

Die Mastitis bei Wiederkäuern wird im Wesentlichen in drei Stadien eingeteilt:
subklinische, chronische und akute Mastitis. Die subklinische Mastitis zeigt keine Veränderungen des Eutersekretes oder Palpationsbefunde, lediglich bei der Milchleistungsprüfung wird eine erhöhte somatische Zellzahl von >250.000/ml festgestellt. International sind die Forderung der Zellzahl bei der Milchleistungsprüfung sehr unterschiedlich von <200.000/ml bis <750.000/ml.

Typisch für die chronische Mastitis sind die bindegewebigen palpierbaren Veränderungen im Drüsengewebe. Das Eutersekret behält Milchcharakter enthält aber Eiterflocken. Die akute Mastitis zeigt typische Entzündungszeichen wie Wärme, Rötung und Schmerzhaftigkeit am erkrankten Euterviertel. Das Tier zeigt Fieber. Das Sekret behält Milchcharakter, enthält aber zusätzlich gelbliche Eiter- und/oder weißliche Fibrinflocken.

Die typischen Erreger der Mastitis sind Staphylococcus aureus, Streptococcus agalactiae, Streptococcus dysgalactiae, Pseudomonas, Proteus, E. Coli und andere oder Hefen.

Die Behandlung von Mastitis wird in der Regel mit Antibiotika durchgeführt. Allerdings ist der Therapierfolg aufgrund der Entwicklung multipler Resistenzen einiger bakterieller Erreger gesunken. Die Behandlungsmöglichkeiten durch Antibiotika sind in letzter Zeit verringert.

Die Milch von Kühen, die bei chronischer oder akuter Mastitis mit Antibiotika behandelt werden, darf nicht in den Handel gelangen und muss deshalb entsorgt werden. Der weltweite Schaden in der Milchwirtschaft wird auf 35 Milliarden USD geschätzt.

Um die Resistenzentwicklungen der pathogenen Bakterien zu reduzieren ist es notwendig alternative effiziente Behandlungsmethoden zu verwenden, so dass auf eine Behandlung mit Antibiotika verzichtet werden kann. Des Weiteren sollten Behandlungen mit Antibiotika vermieden werden, um auszuschließen, das absichtlich oder unabsichtlich Milch von Antibiotika behandelten Kühen zum menschlichen Konsum in den Umlauf gebracht werden.

Alternative homöopathische Therapien zur Behandlung der Mastitis sind wirtschaftlich nicht sinnvoll einsetzbar.

Aus WO 2016/097755 A1 ist bekannt, Carvacrol, Thymol, Curcumin und Piperidin in Kombination mit eine Antibiotikum (Aminoglycosid) zur Behandlung unter anderem auch von Mastitis zu verwenden.

Aus WO 97/01348 A1 ist bekannt, eine Zusammensetzung aus Pflanzenöl, Thymol und Carvacrol zur Behandlung unter anderem von Mastitis zu verwenden, wobei das Verhältnis von Carvacrol zu Thymol wenigstens 10 betragen soll.

WO 97/07812 A1 ist bekannt, eine Zusammensetzung aus Carvacrol und Thymol zur Behandlung von Mastitis zu verwenden, wobei das Verhältnis von Carvacrol zu Thymol wenigstens 20 betragen soll.

Aus FRATINI FILIPPO ET AL. "Antibacterial acitivity of essential oils, their blends and mixtures of their main constituents against some strains supporting livestock mastitis" FITOTERPIA, IDB HOLDING, MILAN, IT, Bd. 96, 13. April 2014, Seiten 1 - 7, XP028873716; ISSN: 0367-326X, DOI: 10.1016/J.FITOTE:2014.04.003, ist bekannt, dass nicht nur Carvacrol und Thymol in einem Gesmisch zu einer guten Wirkung bei der Behandlung von Mastitis führen, sondern dass auch andere Hauptkomponenten zu einem synergetischen Effekt hinsichtlich der beobachteten Aktivität führen können.

Aus MUSHTAQ SALEEM ET AL.: "Bovine mastitis: An appraisal of its alternative herbal cure", MICROBIAL PATHOGENESIS, ACADEMIC PRESS LIMITED, NEW YORK, NY, US, Bd. 114, 9. Dezember 2017, Seiten 357361, XP085349481, ISSN:0882-4010, DOI: 10.1016/J.MICPATH.2017.12.024,ist bekannt, ein Gemisch aus Thymol und Cinnamaldehyd zur Behandlung von Mastitis zu verwenden. Es wird aber auch offenbart, dass zukünftig umfassende Untersuchungen durchgeführt werden müssen, um das große medizinische Potential verschiedener Pflanzenspezies zu entdecken.

Der Erfindung liegt deshalb die Aufgabe zugrunde, alternative Wirkstoffe zur Verwendung für die Behandlung von Mastitis in einer optimierten Weise vorzustellen.

Die Aufgabe wird gelöst durch einen Wirkstoff zur Verwendung für die Behandlung von Mastitis, der dieBestandteile Curcumin und/oder Curcuminoide, Carvacrol, Thymol, P-Cymol, Cinnamaldehyd und Piperine aufweist.

Die Bestandteile können zumindest teilweise Pflanzenextrakte natürlichen Ursprungs sein. Sie können aber auch zumindest teilweise synthetisch hergestellt sein. Letzteres hat den Vorteil immer gleichbleibender Konzentration und Reinheit.

Extrakte aus Pflanzen sind erheblichen Schwankungen in ihrer Zusammensetzung ausgesetzt. Der Ort - die Klimazone, die Jahreszeit, die Tageszeit der Ernte, Bodenfeuchtigkeit, Sonneneinstrahlung, Düngung, mikrobiologischer oder Insektenbefall, Art der Extraktion und die genetischen Varianten innerhalb einer Spezies haben einen großen Einfluss auf die gesamte Konzentration und die Komposition der wirksamen Phytobiotika in den Extrakten. Aus diesem Grunde variieren die Behandlungserfolge dieser Pflanzen-extrakte, wie vielfach in der Literatur dokumentiert. Bei Produkten aus Pflanzenextrakten zur Behandlung von Mastitis wird lediglich die Konzentration der Spezies angegeben, aber es zweifelhaft ob es sich um die gleiche Pflanzenspezies mit den gleichen genetischen oder epigenetischen Charakteristika handelt, wenn sie in zum Beispiel in zwei völlig verschieden Klimazonen angesiedelt sind. Bei der Verwendung von natürlichen Pflanzenextrakten sollte deshalb vorzugsweise eine Konzentration von 90-99% erreicht werden.

Vorteilhaft können die gewichtsbezogenen Verhältnisse der Anteile von Curcumin und/oder Curcuminoiden zu den anderen Bestandteilen in den folgenden Größenordnungen liegen:

| | |
|---|---|
| Curcumin und/oder Curcuminoide zu Carvacrol: | 100 zu 15 |
| Curcumin und/oder Curcuminoide zu Thymol: | 100 zu 14 |
| Curcumin und/oder Curcuminoide zu P-Cymol: | 100 zu 1 |
| Curcumin und/oder Curcuminoide zu Cinnamaldehyd: | 100 zu 30 |
| Curcumin und/oder Curcuminoiden zu Piperin: | 100 zu 15 |

Die angegebenen Größenordnungen können dabei nur für einzelne Bestandteile oder vorzugsweise für alle Bestandteile gelten.

Die Angabe, dass die Verhältnisse in der jeweils angegebenen Größenordnung liegen sollen, bedeutet, dass Abweichungen von den angegebenen Werten um 20% nach oben oder unten möglich sind.

Bei P-Cymol, englisch p-cymene, soll es sich insbesondere um den Stoff 1-Methyl- 4-ispropylbenzol nach der chemischen Nomenklatur handeln. Es versteht sich von selbst, dass das Molekül insbesondere bei der natürlichen Gewinnung nicht 100% rein vorliegt, sondern zu geringen Anteilen auch weitere Bestandteile in den verwendeten Ausgangsstoffen enthalten sein können.

Der erfindungsgemäße Wirkstoff zur Verwendung für die Behandlung von Mastitis stellt eine synergistische Kombination von Phytobiotika dar und kann zur Behandlung und Vorbeugung von Mastitis bei Säugern ohne Einsatz von Antibiotika als Futterzusatzstoff oder zur äußerlichen Anwendung eingesetzt werden. Gleichzeitig hat sich gezeigt, dass der Wirkstoff die Milchproduktion stimuliert, sowohl vor als auch nach der Geburt.

Insbesondere führt der erfindungsgemäße Wirkstoff zur Verwendung für die Behandlung von Mastitis nicht zu einem Resistenzaufbau der pathogenen Bakterien.

Vorteilhaft kann der Wirkstoff zur oralen Einnahme in Fetten mikroverkapselt sein. Die biologische Verfügbarkeit des Curcumin oder der Curcuminoide wird die Zugabe von Piperinen und die Mikroverkapselung in Fett verbessert, da Fette die Absorption von Curcuminoiden verbessern und Piperine zu einem langsameren Abbau der Curcuminoide führen. Die orale Aufnahme des in Fetten mikroverkapselten Wirkstoffs ist deshalb ausgesprochen vorteilhaft.

Der Wirkstoff kann auch zur äußeren Anwendung in Öl, Creme, Salbe oder eine Emulsion, eingemischt werden, insbesondere in 1-2% Gewichtsanteilen. Er kann dann beispielsweise als Euterdip aufbereitet und eingesetzt werden.

Der Wirkstoff kann auch als Nahrungs- oder Futterzusatz aufbereitet sein. Ein entsprechender Nahrungs- oder Futterzusatz kann den Wirkstoff und weitere Bestandteile wie beispielsweise Füllstoffe enthalten. Vorteilhaft kann der Nahrungs- und Futterzusatz auch Probiotika umfassen. Ergänzend können auch Aromastoffe beigemengt werden, um die Einnahme zu erleichtern. Der Nahrungs- oder Futterzusatz kann insbesondere pulverförmig, pastenförmig oder in Form von Pellets, Tabletten oder Kapseln hergestellt werden.

Der erfindungsgemäße Nahrungs- oder Futterzusatz enthaltend einen Wirkstoff zur Verwendung für die Behandlung von Mastitis kann die Bestandteile des Wirkstoffs insbesondere in folgenden Anteilen enthalten:

| | |
|---|---|
| 1 bis 4 Gew-% | Curcumin und/oder Curcuminoide |
| 0,1 bis 0,6 Gew-% | Carvacrol |
| 0,1 bis 0,6 Gew-% | Thymol |
| 0,01 bis 0,06 Gew-% | P-Cymol |
| 0,3 bis 1,2 Gew-% | Cinnamaldehyd |
| 0,1 bis 0,4 Gew-% | Piperin |

Weiter kann der Futterzusatz Tannine enthalten, insbesondere in einem Anteil von 3 bis 6 Gew-%.

Der erfindungsgemäße Nahrungs- oder Futterzusatz enthaltend einen Wirkstoff zur Verwendung für die Behandlung von Mastitis kann je nach Dosierung vorbeugend und langfristig oder dauerhaft oder auch zur akuten Behandlung im Falle einer Erkrankung eingesetzt werden.

Im Falle der vorbeugenden langfristigen oder dauernden Einnahme hat sich eine Dosierung des erfindungsgemäßen Wirkstoffs von 0,015 g bis 0,04 g pro kg Körpergewicht als sehr wirksam erwiesen.

Für die Behandlung von akuten Erkrankungen ist eine Tagesdosis des Wirkstoffs von 0,08 g bis 0,35 g pro kg Körpergewicht während einer Dauer von 5 bis 14 Tagen wirksam.

In Untersuchungen im Labor konnte eine wachstumshemmende bis abtötende Wirkung der erfindungsgemäßen Formulierung in verschiedenen pathogenen Bakterien beobachtet werden. Die Wachstumshemmung bis hin zur bakteriziden Wirkung erfolgt durch eine verringerte Membranstabilitat, Veränderung von Membranproteinen, einer Reduzierung der Replikationsrate und durch eine Hemmung des Quorum-Sensing der pathogenen Bakterien.

In Versuchen konnte bei einer täglichen Einnahme von 1-10g der erfindungsgemäßen Formulierung pro Säugetier gezeigt werden, dass die Formulierung nur pathogene Bakterienstamme hemmt, aber keine Lactobakterien oder Bifidobakterien, so dass weder die Darm- noch die Haut-Mikrobiota negativ beeinflusst wird. Erst bei einer 100-500fachen Inhibitionskonzentration von Salmonellen und E.Colibakterien ist eine lnhibierung der Lactobakterien und Bifidobakterien zu beobachten.

Im Tier- und in Feldversuchen konnte gezeigt werden, dass die somatische Zellzahl in der Milch der behandelten Kühe durch den erfindungsgemäßen Wirkstoff reduziert werden konnte.

In Feldversuchen konnte gezeigt werden, dass nach einer Dauer von 3-10 Tagen chronische und akute Mastitis in verschieden Wiederkäuern behandelt werden konnte. Weiter wurde dabei die Milchleistung und die Futterverwertung verbessert. Diese Leistung wird nicht von Antibiotika erbracht.

In Tierversuchen konnte gezeigt werden, dass die Milch der mit der erfindungsgemäßen Formulierung behandelten Wiederkäuer höhere Lactoperoxidase Werte aufwiesen. Eine höhere Lactoperoxidase Konzentration verbessert die mikrobiotische Kontrolle der pathogenen Bakterien insbesondere von gram-negativen Bakterien. Diese Leistung wird ebenfalls nicht von Antibiotika erbracht.

Weiter konnte in Tierversuchen gezeigt werden, dass die Milch der mit der erfindungsgemäßen Formulierung behandelten Wiederkäuer bis zu 50% geringere reaktive Sauerstoffspezies (reactive oxygen species - ROS) aufweist. Diese Leistung wird nicht von Antibiotika erbracht.

Die Analyse des Blutes der behandelten Wiederkäuer zeigte eine Verringerung der Leukozyten und der neutrophilen weißen Blutkörperchen, welches mit der Reduzierung der somatischen Zellen in der Milch einherging, was auf eine Kontrolle des Infektionsherdes der Mastitis hindeutet und eine entzündungshemmende Eigenschaft der Erfindung andeutet.

Die Blutserumanalyse der behandelten Wiederkäuer zeigt einen Proteinanstieg, der mit einem Anstieg der Globuline einhergeht.

Der Toll Like Receptor 4 (TLR-4) ist in der Membran von vielen Zellen des Immunsystems zu finden und auch auf den Enterozyten im Darmepithel. TLR-4 aktiviert die Entzündungskaskade bei Kontakt mit Lipo-Poly-Saccharide (LPS) von Gram negativen Bakterien, wie z.B. Escherichia Coli und kann zu typischen Entzündungskrankheiten im Darm führen. Alle Komponenten dieser Erfindung hemmen den TLR-4 und sind damit in der Lage Oberreaktionen des Immunsystems auszugleichen und tragen damit auch zur Kontrolle der entzündlichen Reaktionen der Mastitis bei. Diese Leistung kann von Antibiotika nicht erbracht werden.

Die Erfindung reduziert Botenstoffe wie IL6, TNF-α, Prostaglandine, NF_{K}-B und andere, die Entzündungsreaktionen auslösen oder diese verstärken. Diese Reduktion wird zum einen durch die Modulation der Mikrobiota erzielt, indem die Präsenz von Bifidobakterien gefördert wird und durch direkte Regulierung des Immunsystems. Diese Leistung kann von Antibiotika nicht erbracht werden.

In der äußeren Anwendung der Erfindung werden auch Viren deaktiviert oder zerstört und tragen damit zur schnelleren Wundheilung, zur Verringerung der Entzündungsreaktion und zur Verkürzung der Behandlungsdauer bei. Die Erfindung hat damit ein breiteres Wirkspektrum als Antibiotika, die keinerlei Effekte auf virale Infektionen haben.

Die Erfindung verstärkt die Phagozytose und führt damit zu einer Reduktion der pathogenen Bakterien und damit auch zu einer vermehrten Antigenpräsenz, die T-Zellen und B-Zellen aktiviert. Diese Leistung kann von Antibiotika nicht erbracht werden.

Durch die verstärkte Aktivierung von B-Zellen wird auch die spezifische Antikörperbildung und verbessert damit die körpereigenen Immunantwort auf die pathogenen bakteriellen Erreger der Mastitis. Diese Leistung kann von Antibiotika nicht erbracht werden.

Bei der äußerlichen Anwendung der Erfindung werden pathogene Erreger inaktiviert oder in ihrer Aktivität gehemmt, so dass eine Verbreitung der pathogenen Keime verringert wird.

Die Modulation der Mikrobiota durch die Erfindung führt zu einer Verbesserung der Darmintegrität, beziehungsweise werden Bakterien in ihrem Wachstum stimuliert, die Buttersäure (Butansäure) und andere volatile Fettsauren produzieren.

Die Buttersäure ernährt die Enterozyten und aktiviert die Transkription von Genen, die die "Tight Junction" der Enterozyten verbessern und damit die Durchlässigkeit des Darmepithels verringern und zu einer geringeren Entzündungsreaktion führt.

Die Erfindung führt zu einer Vergrößerung der Darmoberfläche und zu einem besseren Verhältnis von Mikrovilli zu Krypten, die eine bessere Darmintegrität andeutet. Hierdurch wird die Aufnahme der Nahrungsleistung verbessert, was zu einer Verbesserung der Widerstandkraft gegen Mastitis führt.

Bei Wiederkäuern werden die Proteine durch das Rumen Mikrobiom abgebaut, so dass nur wenig dem Futter beigesetztes Protein im Darm absorbiert werden kann. Die optional eingesetzten Tannine fixieren Proteine, die dadurch nicht im Rumen abgebaut werden können, sondern im Darm verfügbar werden. Damit verbessert sich die Proteinverfügbarkeit im Darm was letztlich durch die erhöhte Energieaufnahme zur Verbesserung der Milchleistung beiträgt.

## Patentansprüche

1. Wirkstoff zur Verwendung für die Behandlung von Mastitis, umfassend die folgenden Bestandteile:
- Curcumin und/oder Curcuminoide
- Carvacrol
- Thymol
- P-Cymol
- Cinnamaldehyd
- Piperin

2. Wirkstoff zur Verwendung für die Behandlung von Mastitis nach Anspruch 1, **dadurch gekennzeichnet, dass** die Bestandteile zumindest teilweise synthetisch hergestellt sind.

3. Wirkstoff zur Verwendung für die Behandlung von Mastitis nach Anspruch 1 oder 2, **dadurch gekennzeichnet, dass** das gewichtsbezogene Verhältnis der Anteile von Curcumin und/oder Curcuminioden zu Carvacrol in der Größenordnung von 100 zu 15 liegt.

4. Wirkstoff zur Verwendung für die Behandlung von Mastitis nach einem der Anspruche 1 bis 3, **dadurch gekennzeichnet, dass** das gewichtsbezogene Verhältnis der Anteile von Curcumin und/oder Curcuminioden zu Thymol in der Größenordnung von 100 zu 14 liegt.

5. Wirkstoff zur Verwendung für die Behandlung von Mastitis nach einem der Ansprüche 1 bis 4, **dadurch gekennzeichnet, dass** das gewichtsbezogene Verhältnis der Anteile von Curcumin und/oder Curcuminioden zu **P**- Cymol in der Größenordnung von 100 zu 1 liegt.

6. Wirkstoff zur Verwendung für die Behandlung von Mastitis nach einem der Ansprüche 1 bis 5. **dadurch gekennzeichnet, dass** das gewichtsbezogene Verhältnis der Anteile von Curcumin und/oder Curcuminioden zu Cinnamaldehyd in der Größenordnung von 100 zu 30 liegt.

7. Wirkstoff zur Verwendung für die Behandlung von Mastitis nach einem der Ansprüche 1 bis 6, **dadurch gekennzeichnet, dass** das gewichtsbezogene Verhältnis der Anteile von Curcumin und/oder Curcuminioden zu Piperin in der Größenordnung von 100 zu 15 liegt.

8. Wirkstoff zur Verwendung für die Behandlung von Mastitis nach einem der Ansprüche 1 bis 7, **dadurch gekennzeichnet, dass** der Wirkstoff in Fetten mikroverkapselt ist.

9. Wirkstoff zur Verwendung für die Behandlung von Mastitis nach einem der Ansprüche 1 bis 8, **dadurch gekennzeichnet, dass** der Wirkstoff in 1-2% Gewichtsanteil eingemischt ist in Öl, Creme, Salbe oder eine Emulsion.

10. Wirkstoffzur Verwendung für die Behandlung von Mastitis nach einem der Ansprüche 1 bis 9,
**dadurch gekennzeichnet, dass** eine Tagesdosis des Wirkstoffs von 0,015g bis 0,04 g pro kg Körpergewicht verabreicht wird.

11. Wirkstoffzur Verwendung für die Behandlung von Mastitis nach einem der Ansprüche 1 bis 9, **dadurch gekennzeichnet, dass** eine Tagesdosis des Wirkstoffs von 0,08 g bis 0,35 g pro kg Körpergewicht während einer Dauer von 5 bis 14 Tagen verabreicht wird.

12. Nahrungs- oder Futterzusatz zur Verwendung zur Behandlung von Mastitis enthaltend einen Wirkstoff zur Verwendung nach einem der Ansprüche 1 bis 11.

13. Nahrungs- oder Futterzusatz zur Verwendung nach Anspruch 12, **dadurch gekennzeichnet, dass** die Bestandteile des Wirkstoffs in folgenden Anteilen im Nahrungs- oder Futterzusatz enthalten sind:
| | |
|---|---|
| 1 bis 4 Gew-% | Curcumin und/oder Curcuminoide |
| 0,1bis 0,6 Gew-% | Carvacrol |
| 0,1 bis 0,6 Gew-% | Thymol |
| 0,01 bis 0,06 Gew-% | P-Cymol |
| 0,3 bis 1,2 Gew-% | Cinnamaldehyd |
| - 0,1 bis 0,4 Gew-% | Piperin |
und der Nahrungs- oder Futterzusatz darüber hinaus Probiotika und/oder Füllstoffe umfasst.

14. Nahrungs- oder Futterzusatz zur Verwendung nach Anspruch 12 oder 13, **dadurch gekennzeichnet, dass** dieser Tannine enthält, insbesondere in einem Anteil von 3 bis 6 Gew-%.

## Claims

1. An active substance for use in the treatment of mastitis, comprising the following constituents:
- curcumin and/or curcuminoids
- carvacrol
- thymol
- P-cymene
- cinnamaldehyde
- piperine.

2. The active substance for use in the treatment of mastitis according to claim 1, **characterised in that** the constituents are produced at least partially synthetically.

3. The active substance for use in the treatment of mastitis according to claim 1 or 2, **characterised in that** the weight-based ratio of the proportions of curcumin and/or curcuminoids to carvacrol lies in the order or magnitude of 100 to 15.

4. The active substance for use in the treatment of mastitis according to any one of claims 1 to 3, **characterised in that** the weight-based ratio of the proportions of curcumin and/or curcuminoids to thymol lies in the order or magnitude of 100 to 14.

5. The active substance for use in the treatment of mastitis according to any one of claims 1 to 4, **characterised in that** the weight-based ratio of the proportions of curcumin and/or curcuminoids to P-cymene lies in the order or magnitude of 100 to 1.

6. The active substance for use in the treatment of mastitis according to any one of claims 1 to 5, **characterised in that** the weight-based ratio of the proportions of curcumin and/or curcuminoids to cinnamaldehyde lies in the order or magnitude of 100 to 30.

7. The active substance for use in the treatment of mastitis according to any one of claims 1 to 6, **characterised in that** the weight-based ratio of the proportions of curcumin and/or curcuminoids to piperine lies in the order or magnitude of 100 to 15.

8. The active substance for use in the treatment of mastitis according to any one of claims 1 to 7, **characterised in that** the active substance is micro-encapsulated in fats.

9. The active substance for use in the treatment of mastitis according to any one of claims 1 to 8, **characterised in that** the active substance is mixed in a 1-2% weight proportion into oil, cream, ointment or an emulsion.

10. The active substance for use in the treatment of mastitis according to any one of claims 1 to 9, **characterised in that** a daily dose of the active substance of from 0.015 g to 0.04 g per kg of body weight is administered.

11. The active substance for use in the treatment of mastitis according to any one of claims 1 to 9, **characterised in that** a daily dose of the active substance of from 0.08 g to 0.35 g per kg of body weight is administered for a duration of from 5 to 14 days.

12. A food additive or feed additive for use in the treatment of mastitis, containing an active ingredient for the use according to any one of claims 1 to 11.

13. The food additive or feed additive for use according to claim 12, **characterised in that** the constituents of the active substance are contained in the food additive or feed additive in the following proportions:
| | |
|---|---|
| 1 to 4 wt.% | curcumin and/or curcuminoids |
| 0.1 to 0.6 wt.% | carvacrol |
| 0.1 to 0.6 wt.% | thymol |
| 0.01 to 0.06 wt.% | P-cymene |
| 0.3 to 1.2 wt.% | cinnamaldehyde |
| 0.1 to 0.4 wt.% | piperine |
and the food additive or feed additive additionally comprises probiotics and/or fillers.

14. The food additive or feed additive for use according to claim 12 or 13, **characterised in that** it contains tannins, in particular in a proportion of from 3 to 6 wt.%.

## Revendications

1. Principe actif, destiné à une utilisation pour le traitement de la mastite, comprenant les composants suivants :
- des curcumines et/ou des curcuminoïdes
- du carvacrol
- du thymol
- du p-cymène
- du cinnamaldéhyde
- de la pipérine

2. Principe actif, destiné à une utilisation pour le traitement de la mastite selon la revendication 1, **caractérisé en ce que** les composants sont de fabrication au moins partiellement synthétique.

3. Principe actif, destiné à une utilisation pour le traitement de la mastite selon la revendication 1 ou 2, **caractérisé en ce que** le rapport en poids des parts de curcumine et/ou de curcuminoïdes au carvacrol se situe dans l'ordre de grandeur de 100 à 15.

4. Principe actif, destiné à une utilisation pour le traitement de la mastite selon l'une quelconque des revendications 1 à 3, **caractérisé en ce que** le rapport en poids des parts de curcumine et/ou de curcuminoïdes au thymol se situe dans l'ordre de grandeur de 100 à 14.

5. Principe actif, destiné à une utilisation pour le traitement de la mastite selon l'une quelconque des revendications 1 à 4, **caractérisé en ce que** le rapport en poids des parts de curcumine et/ou de curcuminoïdes au p-cymène se situe dans l'ordre de grandeur de 100 à 1.

6. Principe actif, destiné à une utilisation pour le traitement de la mastite selon l'une quelconque des revendications 1 à 5, **caractérisé en ce que** le rapport en poids des parts de curcumine et/ou de curcuminoïdes au cinnamaldéhyde se situe dans l'ordre de grandeur de 100 à 30.

7. Principe actif, destiné à une utilisation pour le traitement de la mastite selon l'une quelconque des revendications 1 à 6, **caractérisé en ce que** le rapport en poids des parts de curcumine et/ou de curcuminoïdes à la pipérine se situe dans l'ordre de grandeur de 100 à 15.

8. Principe actif, destiné à une utilisation pour le traitement de la mastite selon l'une quelconque des revendications 1 à 7, **caractérisé en ce que** le principe actif est microencapsulé dans des graisses.

9. Principe actif, destiné à une utilisation pour le traitement de la mastite selon l'une quelconque des revendications 1 à 8, **caractérisé en ce que** le principe actif est mélangé à raison d'une part en poids de 1 à 2 % dans une huile, une crème, une pommade ou un émulsion.

10. Principe actif, destiné à une utilisation pour le traitement de la mastite selon l'une quelconque des revendications 1 à 9, **caractérisé en ce que** l'on administre une dose journalière du principe actif de 0,015 g à 0,04 g par kg de poids corporel.

11. Principe actif, destiné à une utilisation pour le traitement de la mastite selon l'une quelconque des revendications 1 à 9, **caractérisé en ce que** l'on administre une dose journalière du principe actif de 0,08 g à 0,35 g par kg de poids corporel pendant une durée de 5 à 14 jours.

12. Additif alimentaire ou additif alimentaire pour animaux, destiné à une utilisation pour le traitement de la mastite, contenant un principe actif destiné à une utilisation selon l'une quelconque des revendications 1 à 11.

13. Additif alimentaire ou additif alimentaire pour animaux, destiné à une utilisation selon la revendication 12, **caractérisé en ce que** les composants du principe actif sont contenus selon les parts suivantes dans l'additif alimentaire ou l'additif alimentaire pour animaux :
| | |
|---|---|
| de 1 à 4 % en poids de | curcumine et/ou de curcuminoïdes |
| de 0,1 à 0,6 % en poids de | carvacrol |
| de 0,1 à 0,6 % en poids de | thymol |
| de 0,01 à 0,06 % en poids de | p-cymène |
| de 0,3 à 1,2 % en poids de | cinnamaldéhyde |
| de 0,1 à 0,4 % en poids de | pipérine |
et **en ce que** l'additif alimentaire ou additif alimentaire pour animaux comprend de préférence des probiotiques et/ou des agents de charge.

14. Additif alimentaire ou additif alimentaire pour animaux, destiné à une utilisation selon la revendication 12 ou 13, **caractérisé en ce que** ce dernier contient des tannins, notamment dans une part de 3 à 6 % en poids.
